(19) Europäisches Patentamt
European Patent Office
Office européen des brevets

(11) EP 2 270 062 B1

(12) **EUROPEAN PATENT SPECIFICATION**

(45) Date of publication and mention of the grant of the patent:
**05.03.2014 Bulletin 2014/10**

(21) Application number: **10176459.5**

(22) Date of filing: **08.12.2005**

(51) Int Cl.:
***C08G 18/61*** (2006.01) ***C08G 18/67*** (2006.01)
***C08F 290/06*** (2006.01) ***C08F 290/14*** (2006.01)
***G02B 1/04*** (2006.01) ***A61L 27/16*** (2006.01)
***A61L 27/52*** (2006.01)

(54) **Hydrogel copolymers for biomedical devices**

Hydrogelcopolymere für biomedizinische Vorrichtungen

Hydrogel copolymères pour dispositifs biomédicaux

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.12.2004 US 640153 P**

(43) Date of publication of application:
**05.01.2011 Bulletin 2011/01**

(62) Document number(s) of the earlier application(s) in accordance with Art. 76 EPC:
**05853251.6 / 1 841 806**

(73) Proprietor: **Bausch & Lomb Incorporated**
**Rochester, NY 14604 (US)**

(72) Inventors:
- **Lai, Yu-chin**
  **Pittsford, NY 14534 (US)**
- **Lang, Weihong**
  **Penfield, NY 14526 (US)**
- **Quinn, Edmond T.**
  **Rochester, NY 14626 (US)**
- **Ruscio, Dominic V.**
  **Webster, NY 14580 (US)**

(74) Representative: **Vossius & Partner**
**Siebertstrasse 4**
**81675 München (DE)**

(56) References cited:
**WO-A-93/09154**
**WO-A2-2006/026474**
**US-A- 5 352 714**
**US-A- 5 358 995**
**US-A1- 2003 109 637**
**US-A1- 2003 157 141**
**US-A1- 2003 162 862**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 270 062 B1

## Description

### Field of the Invention

The present invention relates to hydrogel copolymers that are useful for forming biomedical devices, particularly ophthalmic devices including contact lenses, intraocular lenses and ophthalmic implants. The copolymers have a desirable combination of oxygen permeability, tensile modulus, and water content, especially for soft contact lenses.

### Background of the Invention

Hydrogels represent a desirable class of materials for the manufacture of various biomedical devices, including ophthalmic devices such as contact lenses. A hydrogel is a hydrated cross-linked polymeric system that contains water in an equilibrium state. Hydrogel lenses offer desirable biocompatibility and comfort. Silicone hydrogels are a known class of hydrogels and are characterized by the inclusion of a silicone-containing material. Typically, a silicone-containing monomer is copolymerized by free radical polymerization with a hydrophilic monomer, with either the silicone-containing monomer or the hydrophilic monomer functioning as a crosslinking agent (a crosslinker being defined as a monomer having multiple polymerizable functionalities) or a separate crosslinker may be employed.

Oxygen permeability is a desirable property for contact lens materials since the human cornea will be damaged if it is deprived of oxygen for an extended period. Oxygen permeability is conventionally expressed in units of barrer, also called Dk. Oxygen transmissibility is a property of contact lens materials related to oxygen permeability. Oxygen transmissibility is oxygen permeability divided by lens thickness, or Dk/t. The contact lens clinical literature has expressed the view that a contact lens should have a Dk/t of at least 87 barrers/mm in order to be worn overnight without corneal swelling, especially when the lens is worn continuously for extended periods of time. The desire for contact lens materials with higher oxygen permeabilities is especially important for thicker contact lenses, such as toric contact lenses, in order to maintain an acceptable Dk/t value.

An advantage of silicone hydrogels over non-silicone hydrogels is that the silicone hydrogels typically have higher oxygen permeability due to the inclusion of the silicone-containing monomer. Stated differently, the oxygen permeability of non-silicone hydrogels is dependent almost exclusively on water content; in contrast, silicone hydrogels contain silicone which is more oxygen permeable than water. Theoretically, a silicone material with no water would make an ideal contact lens material, as pure silicone has an oxygen permeability approximating 600 barrers. However, in practice, pure silicone lenses are very hydrophobic and not wettable by human tears, and tend to adhere to the cornea, making them uncomfortable for longer periods of wear. Generally, a soft contact lens should have a water content of at least 20 weight percent in order to be worn comfortably for extended periods.

Figure 1 is a graph of water content versus oxygen permeability that has been reported in the literature. For conventional, non-silicone hydrogels, the oxygen permeability increases fairly linearly with increasing water content. This is because these conventional hydrogels derive their oxygen permeability almost exclusively on water content. On the other hand, oxygen permeability of silicone hydrogels decreases with increasing water content, at least for water contents ranging from 20 to 50 weight percent.

Figure 1 illustrates the challenge in developing new silicone hydrogel materials. Higher water content may be desired in order to make a contact lens more wettable or comfortable, but higher water content compromises oxygen permeability. In addition, contact lenses with too high tensile modulus may be less comfortable, or even damage the cornea when worn for extended periods.

US 2003/162862 A1 describes a silicone hydrogel formed from a reaction mixture comprising a high molecular weight hydrophilic polymer and an effective amount of an hydroxyl-functionalized silicone-containing monomer.

### SUMMARY OF THE INVENTION

This invention provides hydrogel copolymers that are useful for forming biomedical devices, particularly ophthalmic devices including contact lenses, intraocular lenses and ophthalmic implants. The copolymers have a desirable combination of oxygen permeability, tensile modulus, and water content, especially for soft contact lens applications. Especially, the hydrogel copolymers have higher oxygen permeability for a given water content value, and also exhibit desirable tensile modulus.

The present invention relates to a hydrogel copolymer that is the hydrated polymerization product of a monomeric mixture comprising
a polysiloxane prepolymer comprising soft segments that are a diradical residue of the formula (PS’):

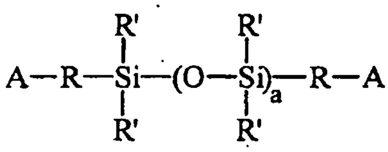

(PS')

wherein:

each A is a hydroxyl or an amino radical;
each R is independently selected from an alkylene group having 1 to 10 carbon atoms wherein the carbon atoms may include ether, urethane or ureido linkages therebetween;
each R' is independently selected from hydrogen, monovalent hydrocarbon radicals or halogen substituted monovalent hydrocarbon radicals wherein the hydrocarbon radicals have 1 to 20 carbon atoms which may include ether linkages therebetween, and
a is at least 1,
wherein the silicon atom content of the prepolymer is at least 32 weight % of the prepolymer;
a monofunctional silicone-containing monomer; and
a hydrophilic comonomer selected from the group consisting of: methacrylic acid; acrylic acid; 2-hydroxyethylmethacrylate; N-vinyl pyrrolidone; and methacrylamide; the hydrogel copolymer having a water content in the range of 40 to 50 weight percent, and an oxygen permeability greater than 100 barrers.

The above hydrogel copolymer may have either a water content in the range of 40 to no greater than 45 weight percent, or a water content in the range of 45 to 50 weight percent.

The above hydrogel copolymer may have a modulus between about 40 and 80 g/mm$^2$.

Furthermore, the present invention relates to a contact lens made of the above hydrogel copolymer.

Herein described, but not related to the claimed invention, is also a hydrogel copolymer of either: a water content in the range of at least 20 to no greater than 40 weight percent, and an oxygen permeability greater than (330-6x) barrers, more preferably (350-6x) barrers, where x has a value equal to weight percent water content; or a water content in the range of 40 to no greater than 45 weight percent, and an oxygen permeability greater than 80 barrers; or a water content in the range of 45 to 50 weight percent, and an oxygen permeability greater than 70 barrers.

Herein described, but not related to the claimed invention, is also a hydrogel copolymer having a water content in the range of at least 20 to no greater than 40 weight percent, and an oxygen permeability greater than (380-6x) barrers. More preferably, the hydrogel copolymer has a water content in the range of at least 20 to no greater than 40 weight percent, and an oxygen permeability greater than (410-6x) barrers. Most preferably, the hydrogel copolymer has a water content in the range of at least 20 to no greater than 40 weight percent, and an oxygen permeability greater than (440-6x) barrers.

Herein described, but not related to the claimed invention, is also a hydrogel copolymer which is the hydrated polymerization product of a monomeric mixture comprising a polysiloxane prepolymer and a hydrophilic comonomer, the hydrogel copolymer having either: (i) a water content in the range of at least 20 to no greater than 30 weight percent, and an oxygen permeability of at least 200 barrers; (ii) a water content in the range of at least 30 to no greater than 40 weight percent, and an oxygen permeability greater than 150 barrers; or (iii) a water content in the range of 40 to 50 weight percent, and an oxygen permeability greater than 100 barrers.

Preferably, the hydrogel copolymers have a modulus no greater than 100 g/mm$^2$, especially between about 40 and 80 g/mm$^2$. In the present invention, the silicon atom content of the prepolymer is at least 32% of the prepolymer, and preferably at least 33% of the prepolymer. Preferably, the prepolymer has a molecular weight (Mn) of at least 10,000, especially at least 15,000, and more preferably, at least 20,000.

Herein described, but not related to the claimed invention, is also a preferred class of hydrogel copolymers having a modulus no greater than 100 g/mm$^2$, an oxygen permeability of at least 140 barrers, and a water content of at least 25 weight percent. An especially preferred class of hydrogel copolymers has a modulus between about 40 and 80 g/mm$^2$, an oxygen permeability of at least 200 barrers, and a water content of at least 25 weight percent.

Herein described, but not related to the claimed invention, is also an embodiment including a hydrogel copolymer that is the hydrated polymerization product of a monomeric mixture comprising a polysiloxane prepolymer and a hydrophilic comonomer, the hydrogel copolymer having a water content of at least 40 weight percent, and an oxygen permeability greater than 70 barrers.

Preferred silicone hydrogel copolymers are the hydrated polymerization product of a monomeric mixture comprising a polysiloxane prepolymer, a vinyl lactam, such as N-vinylpyrollidone, and a (meth)acrylated alcohol, such as 2-hydroxyethylmethacrylate or glyceryl methacrylate.

This invention further provides a biomedical device comprised of the copolymer, especially an ophthalmic device such as a contact lens or an intraocular lens.

BRIEF DESCRIPTION OF THE DRAWINGS

Figure 1 is a graph of water content versus oxygen permeability, illustrating the effect of water content on Dk for conventional silicone hydrogels and non-silicone hydrogels.

Figure 2 is a graph of water content versus oxygen permeability for various prior silicone hydrogels and the silicone hydrogels of this invention.

DETAILED DESCRIPTION OF PREFERRED EMBODIMENTS

Figure 2 is a graph of water content (weight percent water) versus oxygen permeability (barrer). The line in Figure 2 was developed empirically by applicant by measuring and correlating various prior silicone hydrogel copolymers. As seen in Figure 2, for water contents ranging from 20 to about 35 weight percent, the line is relatively linear with a slope approximating minus 6 (-6). In the range of 35 to 40 weight percent, the line begins to plateau. This line graphed in Figure 2 actually correlates well with the reported water contents and oxygen permeabilities of four commercially available silicone hydrogel contact lenses, represented by the circled points in Figure 2: lotrafilcon A (sold under the trademark Focus Night & Day by CIBA Vision Corporation) having Dk of 140 barrers and 24% water; lotrafilcon B (sold under the trademark 02 Optics by CIBA Vision Corporation) having Dk of 110 and 33% water; balafilcon A (sold under the trademark PureVision by Bausch & Lomb Incorporated) having Dk of 91 and 36% water; and galyfilcon A (sold under the trademark Acuvue Advance by Johnson & Johnson Vision Care, Inc.) having Dk of 60 barrers and 47 % water.

The points on Figure 2 falling well above the line are various hydrogel copolymers reported in the examples of this application.

The hydrogel copolymers of the present invention are the hydrated polymerization product of a monomeric mixture comprising a polysiloxane prepolymer, a hydrophilic comonomer, and a monofunctional silicone-containing monomer.

The polysiloxane prepolymers include polysiloxane-containing soft segments. These segments are derived from polysiloxanes endcapped with hydroxyl or amino radicals and represented by the following formula (PS'):

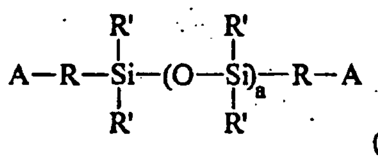

(PS')

wherein each A is a hydroxyl or amino radical;

each R is independently selected from an alkylene group having 1 to 10 carbon atoms wherein the carbon atoms may include ether, urethane or ureido linkages therebetween;

each R' is independently selected from hydrogen, monovalent hydrocarbon radicals or halogen substituted monovalent hydrocarbon radicals wherein the hydrocarbon radicals have 1 to 20 carbon atoms which may include ether linkages therebetween, and

a is at least 1.

Preferred R radicals are alkylene optionally substituted with ether radicals. Preferred R' radicals include: alkyl groups, phenyl groups, fluoro-substituted alkyl groups and alkenyl groups, optionally substituted ether groups. Especially preferred R' radicals include: alkyl, such as methyl; or fluoroalkyl optionally including ether linkages, such as $-CH_2-CH_2-CH_2-O-CH_2-(CF_2)_z-H$ where z is 1 to 6.

Preferably, a is about 10 to about 100, more preferably about 15 to about 80. The Mn of PS ranges from 1000 to 8000, more preferably 2000 to 6000.

Various polysiloxane-diols and polysiloxane-diamines are commercially available. Additionally, representative syntheses of polysiloxanes are provided in the Examples.

The silicon atom content of the prepolymer is at least 32 weight % of the prepolymer, and preferably at least 33 weight % of the prepolymer. Silicon atom content is defined as the total weight of silicon atoms in the prepolymer, per total weight of the prepolymer, times 100%.

The prepolymers are endcapped at both ends with a polymerizable ethylenic unsaturated radical. Preferred

terminal polymerizable radicals are represented by formula (M'):

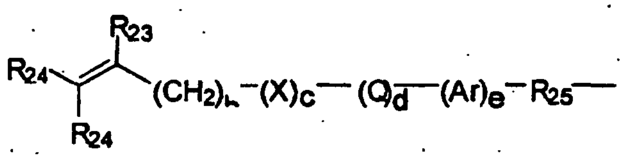

(M')

wherein:

$R_{23}$ is hydrogen or methyl;
each $R_{24}$ is hydrogen, an alkyl radical having 1 to 6 carbon atoms, or a -CO-Y-$R_{26}$ radical wherein Y is -O-, -S- or -NH-;
$R_{25}$ is a divalent alkylene radical having 1 to 10 carbon atoms;
$R_{26}$ is a alkyl radical having 1 to 12 carbon atoms;
Q denotes -CO-, -OCO- or -COO-;
X denotes -O- or -NH-;
Ar denotes an aromatic radical having 6 to 30 carbon atoms; b is 0 to 6; c is 0 or 1; d is 0 or 1; and e is 0 or 1.

Suitable endcapping precursors, for forming the M radicals, include: hydroxy-terminated (meth)acrylates, such as 2-hydroxyethylmethacrylate, 2-hydroxyethylacrylate, and 3-hydroxypropylmethacrylate; and amino-terminated (meth) acrylates, such as t-butylaminoethylmethacrylate and aminoethylmethacrylate; and (meth)acrylic acid. (As used herein, the term "(meth)" denotes an optional methyl substituent. Thus, terms such as "(meth)acrylate" denotes either methacrylate or acrylate, and "(meth)acrylic acid" denotes either methacrylic acid or acrylic acid.)
Preferably, the prepolymer has a molecular weight (Mn) of at least 10,000, more preferably at least 15,000, and most preferably at least 20,000.
The copolymers of this invention are formed by copolymerizing the polysiloxane prepolymers with one or more comonomers. Since the prepolymers are endcapped with polymerizable ethylenically unsaturated radicals, they are polymerizable by free radical polymerization. The monomeric mixtures employed in the invention include conventional lens-forming or device-forming monomers. (As used herein, the term "monomer" or "monomeric" and like terms denote relatively low molecular weight compounds that are polymerizable by free radical polymerization, as well as higher molecular weight compounds also referred to as "prepolymers", "macromonomers", and related terms.) For copolymers, the subject prepolymers are included in the monomer mixture at 5 to 95 weight percent, preferably 20 to 70 weight percent.
At least one hydrophilic comonomer is combined with the polysiloxane prepolymer in the initial monomeric mixture. The monomeric mixture used in the present invention comprises a hydrophilic comonomer selected from the group consisting of methacrylic acid; acrylic acid; 2-hydroxyethyl-methacrylate; N-vinyl pyrrolidone; and methacrylamide. Representative hydrophilic comonomers include: unsaturated carboxylic acids, such as methacrylic and acrylic acids; (meth)acrylic substituted alcohols, such as 2-hydroxyethylmethacrylate, 2-hydroxyethylacrylate and glyceryl methacrylate; vinyl lactams, such as N-vinyl pyrrolidone; and (meth)acrylamides, such as methacrylamide and N,N-dimethylacrylamide. A hydrogel is a crosslinked polymeric system that can absorb and retain water in an equilibrium state. At least one hydrophilic monomer is included in the monomer mixture at 20 to 60 weight percent, preferably 25 to 50 weight percent.
According to various preferred embodiments, the initial monomeric mixture comprises at least one (meth)acrylic substituted alcohol, such as at least one of 2-hydroxyethylmethacrylate and glyceryl methacrylate, preferably in an amount of at least 1 weight percent of the monomeric mixture, more preferably in an amount of 2 to 10 weight percent. Preferably, the monomeric mixture further includes at least one vinyl lactam, such as N-vinyl pyrrolidone and/or at least one (meth)acrylamides, such as N,N-dimethylacrylamide.
Another class of lens-forming or device-forming monomers are silicone-containing monomers. In other words, another silicone-containing comonomer, in addition to the polysiloxane prepolymer, may be included in the initial monomeric mixture, for example, if it is desired to obtain a copolymer with higher oxygen permeability.
The monomeric mixture used in the present invention further comprises a monofunctional silicone-containing monomer.
One suitable class of silicone containing monomers include known bulky, monofunctional polysiloxanylalkyl monomers represented by Formula (VI):

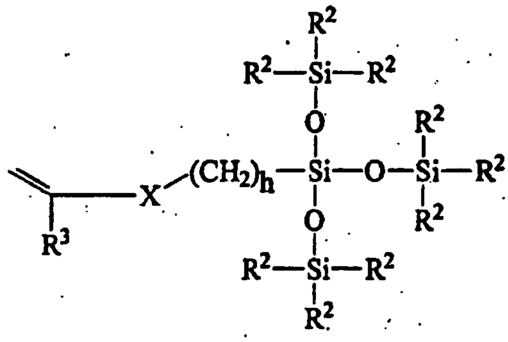

(VI)

X denotes -COO-, -CONR$^4$-, -OCOO-, or -OCONR$^4$- where each where $R^4$ is H or lower alkyl; $R^3$ denotes hydrogen or methyl; h is 1 to 10; and each $R^2$ independently denotes a lower alkyl or halogenated alkyl radical, a phenyl radical or a radical of the formula

$$-Si(R^5)_3$$

wherein each $R^5$ is independently a lower alkyl radical or a phenyl radical. Such bulky monomers specifically include methacryloxypropyl tris(trimethylsiloxy)silane (TRIS), pentamethyldisiloxanyl methylmethacrylate, tris(trimethylsiloxy) methacryloxy propylsilane, methyldi(trimethylsiloxy)methacryloxymethyl silane, 3-[tris(trimethylsiloxy)silyl] propyl vinyl carbamate, and 3-[tris(trimethylsiloxy)silyl] propyl vinyl carbonate.

Various difunctional and multifunctional silicone-containing monomers are known in the art and may be used as a comonomer if desired.

The monomer mixtures may include the silicone comonomer, in addition to the subject prepolymer, at 0 to 50 weight percent, preferably 5 to 30 weight percent when present.

For silicone hydrogels, the monomer mixture includes a crosslinking monomer (a crosslinking monomer being defined as a monomer having multiple polymerizable functionalities). Since the subject prepolymers are endcapped at both ends with a polymerizable radical, the prepolymers will function as a crosslinker. Optionally, a supplemental crosslinking monomer may be added to the initial monomeric mixture. Representative crosslinking monomers include: divinylbenzene, allyl methacrylate, ethyleneglycol dimethacrylate, tetraethyleneglycol dimethacrylate, polyethyleneglycol dimethacrylate, vinyl carbonate derivatives of the glycol dimethacrylates, and methacryloxyethyl vinylcarbonate. When a supplemental crosslinking agent is employed, this monomeric material may be included in the monomer mixture at 0.1 to 20 weight percent, more preferably at 0.2 to 10 weight percent.

In the case of intraocular lenses, the monomer mixtures may further include a monomer for increasing the refractive index of the resultant copolymer. Examples of such monomers are aromatic (meth) acrylates, such as phenyl (meth)acrylate, phenylethyl (meth)acrylate and benzyl (meth)acrylate.

An organic diluent may be included in the initial monomeric mixture. As used herein, the term "organic diluent" encompasses organic compounds that are substantially unreactive with the components in the initial mixture, and are often used to minimize incompatibility of the monomeric components in this mixture. Representative organic diluents include: monohydric alcohols, such as $C_2$-$C_{10}$ monohydric alcohols; diols such as ethylene glycol; polyols such as glycerin; ethers such as diethylene glycol monoethyl ether; ketones such as methyl ethyl ketone; esters such as methyl heptanoate; and hydrocarbons such as toluene.

In forming lenses or other biomedical devices, the monomeric mixtures may be charged to a mold, and then subjected to heat and/or light radiation, such as UV radiation, to effect curing, or free radical polymerization, of the monomer mixture in the mold. Various processes are known for curing a monomeric mixture in the production of contact lenses or other biomedical devices, including spincasting and static casting. Spincasting methods involve charging the monomer mixture to a mold, and spinning the mold in a controlled manner while exposing the monomer mixture to light. Static casting methods involve charging the monomer mixture between two mold sections forming a mold cavity providing a desired article shape, and curing the monomer mixture by exposure to heat and/or light. In the case of contact lenses, one mold section is shaped to form the anterior lens surface and the other mold section is shaped to form the posterior lens surface. If desired, curing of the monomeric mixture in the mold may be followed by a machining operation in order to provide a contact lens or article having a desired final configuration. Such methods are described in US Patent Nos. 3,408,429, 3,660,545, 4,113,224, 4,197,266, 5,271,875, and 5,260,000. Additionally, the monomer mixtures may be cast in the shape of rods or buttons, which are then lathe cut into a desired shape, for example, into a lens-shaped article.

Various polysiloxane prepolymers suitable for this invention will now be described.

A first class of polysiloxane prepolymers comprises blocks (I) and (II) and is terminated at each end with an ethylenic unsaturated radical:

$$(*Dii*Diol*Dii*PS)_x \quad (I)$$

$$(*Dii*PS)_y \quad (II)$$

wherein:

each Dii is independently a diradical residue of a diisocyanate;
each Diol is independently a diradical residue of a diol having 1 to 10 carbon atoms;
each PS is independently a diradical residue of a polysiloxane- diol or -diamine;
each * is independently -NH-CO-NH-, -NH-COO- or -OCO-NH-:

x represents the number of blocks (I) and is at least 2, and
y represents the number of blocks (II) and is at least 1.

This class of prepolymers includes those represented by the general formulae:

$$M(*Dii*Diol*Dii*PS)_x(*Dii*PS)_y*Dii*M \quad (III)$$

or

$$M(*Dii*Diol*Dii*PS)_x(*Dii*PS)_y*Dii*Diol*Dii*M \quad (IV)$$

wherein Dii, Diol, PS, *, x and y are as defined above, and M is a polymerizable ethylenically unsaturated radical.

Generally, the blocks of formula (I) may be characterized as composed of strong hard segments (represented by *Dii*Diol*Dii*) and soft segments (represented by PS). Generally, the blocks of formula (II) may be characterized as composed of weaker hard segments (represented by *Dii*) and soft segments (represented by PS). The distribution of these weaker and strong hard blocks (I) and (II) may be random or alternate, where x and y represent the total number of blocks of respective structures in the prepolymer; stated differently, it is not necessary in formulae (III) and (IV) that all blocks of formula (I) are directly linked to each other. The distribution of these blocks may be controlled by the sequence of addition of the polysiloxane, diisocyanate and short chain diol ingredients during the preparation of the prepolymer.

The prepolymers include polysiloxane-containing soft segments, represented by PS in formulae (I), (II), (III) and (IV). More particularly, this polysiloxane-containing segment is derived from polysiloxanes endcapped with hydroxyl or amino radicals, such as polysiloxane segments represented by formula (PS').

Preferably, a in formula (III) and (IV) is about 10 to about 100, more preferably about 15 to about 80. The Mn of PS ranges from 1000 to 8000, more preferably 2000 to 6000.

The strong hard segments of the prepolymers include the residue of a diol, represented by Diol in formulae (I), (III) and (IV). Preferred Diol radicals include the diradical residue of an alkyl diol, a cycloalkyl diol, an alkyl cycloalkyl diol, an aryl diol or an alkylaryl diol having 1 to 10 carbon atoms and which may contain ether, thio or amine linkages in the main chain. Representative diols include 2,2-(4,4'-dihydroxydiphenyl)propane (bisphenol-A), 4,4'-iso-propylidine dicyclohexanol, ethoxylated and propoxylated bisphenol-A, 2,2-(4,4'-dihydroxydiphenyl)pentane, 1,1'-(4,4'-dihydroxydiphenyl)-p-diisopropyl benzene, 1,3-cyclohexane diol, 1,4-cyclohexane diol, 1-4-cyclohexane dimethanol, neopentyl glycol, 1,4-butanediol, 1,3-propanediol, 1,5-pentanediol, ethylene glycol, diethylene glycol and triethylene glycol. Especially preferred are alkylene and etherified alkylene diols having 1 to 10 carbon atoms.

The aforementioned polysiloxane-containing segments and diol residue segments are linked via diisocyanates that react with hydroxyl- or amino-functionality of the polysiloxane-containing segments and diols. Generally, any diisocyanate may be employed. These diisocyanates may be aliphatic or aromatic, and include alkyl, alkyl cycloalkyl, cycloalkyl, alkyl aromatic and aromatic diisocyanates preferably having 6 to 30 carbon atoms in the aliphatic or aromatic moiety. Specific examples include isophorone diisocyanate, hexamethylene-1,6- diisocyanate, 4,4'-dicyclohexylmethane

diisocyanate, toluene diisocyanate, 4,4'-diphenyl diisocyanate, 4,4'-diphenylmethane diisocyanate, p-phenylene diisocyanate, 1,4-phenylene 4,4'-diphenyl diisocyanate, 1,3-bis-(4,4'-isocyanto methyl) cyclohexane, and cyclohexane diisocyanate.

Generally, higher x values results in prepolymers have a higher number of polar urethane/urea linkages, and polarity of the prepolymer is important to ensure compatibility with hydrophilic co-monomers. Generally, higher y values results in prepolymers with a higher percentage of silicon, resulting in higher oxygen permeability. However, the ratio of x and y should be balanced. Accordingly, the ratio of x to y is preferably at least 0.6 (i.e., x:y is at least 0.6:1), more preferably at least 0.75.

The prepolymers are endcapped at both ends with a polymerizable ethylenic unsaturated radical, represented by M in formulae (III) and (IV). Representative M radicals are represented by formula (M').

A first representative reaction scheme for forming the prepolymers is as follows. First, a diisocyanate is reacted with a diol, at a molar ratio of 2:1, respectively.

$$2x\ \mathrm{OCN\text{-}Dii\text{-}NCO} + x\ \mathrm{HO\text{-}Diol\text{-}OH} \rightarrow x\ \mathrm{OCN\text{-}Dii{*}Diol{*}Dii\text{-}NCO}$$

In this scheme, * designates a urethane radical -NH-COO- or -OCO-NH-: Generally, this reaction is conducted in the presence of a catalyst, such as dibutyl tin dilaurate and in a solvent, such as methylene chloride, and under reflux. Then, a diisocyanate and the polysiloxane-diol are added, with the ratio of total diisocyanates (x+y) to polysiloxane-diol being at least 1.1. (Generally, $2 < x+y \leq 11$; $x > 0$; $y > 0$.)

$$x\ \mathrm{OCN\text{-}Dii\text{-}{*}\text{-}Diol\text{-}{*}\text{-}Dii\text{-}NCO} + (x+y-1)\ \mathrm{HO\text{-}PS\text{-}OH} + y\ \mathrm{OCN\text{-}Dii\text{-}NCO} \rightarrow$$
$$\mathrm{OCN\text{-}(Dii{*}Diol{*}Dii{*}PS)_x({*}Dii{*}PS)_y{*}Dii\text{-}NCO}$$

Finally, this product is endcapped with the polymerizable ethylenically unsaturated radical.

$$\mathrm{OCN\text{-}Dii{*}Diol{*}Dii{*}PS)_x({*}Dii{*}PS)_y{*}Dii\text{-}NCO} + 2\ \mathrm{M\text{-}OH} \rightarrow$$
$$\mathrm{M({*}Dii{*}Diol{*}Dii{*}PS)_x({*}Dii{*}PS)_y{*}Dii{*}M}$$

A second representative reaction scheme for forming the prepolymers of formula (I), (II), (III) and/or (IV) is as follows. First, a diisocyanate is reacted with the polysiloxane-diol at a molar ratio shown below, where (1+1/m) preferably ranges from 1.05 to 1.9, most preferably from 1.2 to 1.5.

$$(m+1)\ \mathrm{OCN\text{-}Dii\text{-}NCO} + m\ \mathrm{HO\text{-}PS\text{-}OH} \rightarrow \mathrm{OCN\text{-}(Dii{*}PS)_m{*}Dii\text{-}NCO}$$

In this scheme, * again designates a urethane radical -NH-COO- or -OCO-NH-. Generally, this reaction is conducted in the presence of a catalyst, such as dibutyl tin dilaurate and in a solvent, such as methylene chloride, and under reflux. Then, the diol is added, with the molar ratio selected based on the desired ratio of strong and weak hard segments, with reflux continued, where z1/z2 is equal to or lower than 2 but higher than 1.

$$z1\ \mathrm{OCN\text{-}(Dii\text{-}{*}\text{-}PS)_m\text{-}{*}\text{-}Dii\text{-}NCO} + z2\ \mathrm{HO\text{-}Diol\text{-}OH} \rightarrow$$
$$\mathrm{OCN\text{-}Dii{*}Diol{*}Dii{*}PS)_x({*}Dii{*}PS)_y{*}Dii\text{-}NCO}$$

Finally, this product is endcapped with the polymerizable ethylenically unsaturated radical.

$$\mathrm{OCN\text{-}Dii{*}Diol{*}Dii{*}PS)_x({*}Dii{*}PS)_y{*}Dii\text{-}NCO} + 2\ \mathrm{M\text{-}OH} \rightarrow$$
$$\mathrm{M({*}Dii{*}Diol{*}Dii{*}PS)_x({*}Dii{*}PS)_y{*}Dii{*}M}$$

In the above reaction schemes, the reaction of diols with diisocyanates yields urethane radicals (-NH-COO- or -OCO-NH-). Alternatively, the reaction of diamines with diisocyantes would yield urea radicals (-NH-CO-NH-). Other methods for forming urethane or urea polymers are known in the art, and representative syntheses are illustrated in the Examples.

A second class of polysiloxane prepolymers are represented by the formula:

$$M(*Dii*\ PS)_x\ *Dii*M \qquad (V)$$

wherein:

Dii, PS, * and M have the same meanings as above. Generally, the *Dii*PS blocks of formula (I) may be characterized as composed of relatively weak hard segments (represented by *Dii*) and soft segments (represented by PS). In formula (V), x is at least two, more preferably at least three.

A representative reaction scheme for forming this class of prepolymers is as follows. First, a diisocyanate is reacted with the polysiloxane-diol.

$$(n+1)\ OCN\text{-}Dii\text{-}NCO\ +\ n\ HO\text{-}PS\text{-}OH\ \rightarrow\ OCN\text{-}(Dii*PS)x*Dii\text{-}NCO$$

In this scheme, * designates a urethane radical -NH-COO- or -OCO-NH-. Generally, this reaction is conducted in the presence of a catalyst, such as dibutyl tin dilaurate and in a solvent, such as methylene chloride, and under reflux.

Finally, this product is endcapped with the polymerizable ethylenically unsaturated radical.

$$OCN\text{-}(Dii*PS)x*Dii\text{-}NCO\ +\ 2\ M\text{-}OH\ \rightarrow$$
$$M(*Dii*\ PS)_x\ *Dii*M$$

In the above reaction scheme, the reaction of the polysiloxane-diol with the diisocyanate yields urethane radicals (-NH-COO- or -OCO-NH-). Alternatively, the reaction of poly-siloxane-diamines with diisocyanates would yield urea radicals (NH-CO-NH-). Other methods for forming urethane or urea polymers are known in the art, and representative syntheses are illustrated in the Examples.

Additional polysiloxane-containing prepolymers are represented by the formulae:

$$M(*Dii*PS*Dii*Diol)_x*Dii*PS*Dii*M \qquad (VI)$$

$$M(*Dii*Diol*Dii*PS)_x*Dii*Diol*Dii*M \qquad (VII)$$

where Dii, PS, Diol, * and Dii have the same meanings as above. In formulae (VI) and (VII), x is at least one. Generally, these prepolymers are composed of alternating strong hard segments (represented by *Dii*Diol*Dii*) and soft segments (represented by PS). These prepolymers may be prepared by methods generally known in the art, including the general methods disclosed in US 5,034,461 (Lai et al.).

For the aforementioned prepolymers containing urethane and/or urea linkages between hard segments and soft segments, it is preferred that the weight ratio of soft segments to hard segments is at least 5:1 1 and no greater than 13:1, and in some cases, preferably at least 7:1 and no greater than 11:1.

The hydrogel copolymer, when fully hydrated, has a water content of at least 20 weight percent, as measured gravimetrically. Espeically preferred are hydrogel copolymers having a water content of at least 25 weight percent.

Also, it is preferred that the hydrogel copolymer has a tensile modulus no greater than 100 g/mm$^2$, more preferably a modulus between about 40 and 80 g/mm$^2$. Modulus may be measured with an Instron (Model 4502) instrument according to ASTM D-1708a, where the hydrogel copolymer film sample is immersed in borate buffered saline. An appropriate size of the film sample is gauge length 22 mm and width 4.75 mm, where the sample further has ends forming a dogbone shape to accommodate gripping of the sample with clamps of the Instron instrument, and a thickness

of 200±50 microns.

It is preferred that the hydrogel copolymer has an oxygen permeability of at least 100 barrers, more preferably at least 140 barrers, most preferably at least 150 barrers. Copolymers having an oxygen permeability of at least 180 barrers, and even at least 200 barrers, are provided by this invention.

The preferred combinations of water content and oxygen permeability may also be described as either (i) a water content in the range of at least 20 to no greater than 40 weight percent, and an oxygen permeability greater than (320-6x) barrers, where x has a value equal to weight percent water content; or (ii) a water content in the range of 40 to 50 weight percent, and an oxygen permeability greater than 70 barrers. Alternatively, these properties may be expressed as either: (i) a water content in the range of at least 20 to no greater than 30 weight percent, and an oxygen permeability of at least 200 barrers; (ii) a water content in the range of at least 30 to no greater than 40 weight percent, and an oxygen permeability greater than 150 barrers; or (iii) as required in the present invention, a water content in the range of 40 to 50 weight percent, and an oxygen permeability greater than 100 barrers.

Oxygen permeability (also referred to as Dk) is determined by the following procedure. Other methods and/or instruments may be used as long as the oxygen permeability values obtained therefrom are equivalent to the described method. The oxygen permeability of silicone hydrogels is measured by the polarographic method (ANSI Z80.20-1998) using an 02 Permeometer Model 201T instrument (Createch, Albany, California USA) having a probe containing a central, circular gold cathode at its end and a silver anode insulated from the cathode. Measurements are taken only on pre-inspected pinhole-free, flat silicone hydrogel film samples of three different center thicknesses ranging from 150 to 600 microns. Center thickness measurements of the film samples may be measured using a Rehder ET-1 electronic thickness gauge. Generally, the film samples have the shape of a circular disk. Measurements are taken with the film sample and probe immersed in a bath containing circulating phosphate buffered saline (PBS) equilibrated at 35°C+/-0.2°. Prior to immersing the probe and film sample in the PBS bath, the film sample is placed and centered on the cathode premoistened with the equilibrated PBS, ensuring no air bubbles or excess PBS exists between the cathode and the film sample, and the film sample is then secured to the probe with a mounting cap, with the cathode portion of the probe contacting only the film sample. For silicone hydrogel films, it is frequently useful to employ a Teflon polymer membrane, e.g., having a circular disk shape, between the probe cathode and the film sample. In such cases, the Teflon membrane is first placed on the pre-moistened cathode, and then the film sample is placed on the Teflon membrane, ensuring no air bubbles or excess PBS exists beneath the Teflon membrane or film sample. Once measurements are collected, only data with correlation coefficient value ($R^2$) of 0.97 or higher should be entered into the calculation of Dk value. At least two Dk measurements per thickness, and meeting $R^2$ value, are obtained. Using known regression analyses, oxygen permeability (Dk) is calculated from the film samples having at least three different thicknesses. Any film samples hydrated with solutions other than PBS are first soaked in purified water and allowed to equilibrate for at least 24 hours, and then soaked in PHB and allowed to equilibrate for at least 12 hours. The instruments are regularly cleaned and regularly calibrated using RGP standards. Upper and lower limits are established by calculating a +/- 8.8% of the Repository values established by William J. Benjamin, et al., The Oxygen Permeability of Reference Materials, Optom Vis Sci 7 (12s): 95 (1997):

| Material Name | Repository Values | Lower Limit | Upper Limit |
|---|---|---|---|
| Fluoroperm 30 | 26.2 | 24 | 29 |
| Menicon EX | 62.4 | 56 | 66 |
| Quantum II | 92.9 | 85 | 101 |

The following Examples 17, 18, 45 and 47 illustrate various preferred embodiments of the invention. The other examples are not within the claims.

### Example 1

**Preparation of α, ω-bis(4-hydroxybutyl)polydimethylsiloxane (Mn about 5000)**

The following were charged to a 2-L , three-neck round-bottom flask equipped with one reflux condenser: 51.26grams of 1,3-bishydroxybutyl tetramethyldisiloxane; 1085 grams of dimethoxydimethylsilane; 157.8 grams of distilled water; and 18.4 mL of concentrated hydrochloric acid. The mixture was heated at 60°C for 1 hour. Methanol was then distilled off over a 5-hour period, with 552 mL collected. Then, 349 ml distilled water and 349 mL concentrated HCl were added, and the contents were refluxed at 100°C for 3 hours. The crude product was then separated from the aqueous layer. Then, 600 mL diethyl ether (ether) and 400 mL deionized water were added, and the solution was

extracted twice with 400 mL sodium bicarbonate solution (0.5 %) and then with distilled water until the washing had neutral pH. The product (655.8 grams) was then added slowly into a mixture of methanol/water (508.2g/147.97 g). The bottom organic layer was separated, added with diethyl ether and dried with magnesium sulfate. Ether was then stripped under vacuum at room temperature and the residue was further stripped under vacuum (9.33 Pa) [0.07-mm torr] at 80° C. The final product was recovered. The molecular weight (Mn) as determined by H-NMR was 4800.

## Examples 2

### Preparation of α, ω-bis(4-hydroxybutyl)polydimethylsiloxane (Mn about 2700)

The general procedure of Example 1 was following for making this polysiloxane, except the molar ratio of 1,3-bishydroxybutyl tetramethyldisiloxane to dimethoxydimethylsilane was changed to about 1:28. The molecular weight (Mn) of the product as determined by titration was 2730.

## Example 3

### Preparation of a polydimethylsiloxane-based prepolymer using PDMS of Example 1 and containing blocks of formulae (I) and (II)

A dry 3-neck, 500-mL round-bottom flask was connected to a nitrogen inlet tube and a reflux condenser. The following were added to the flask all at once: isophorone diisocyanate (2.111 g, 9.497 mmol) (IPDI); diethyleneglycol (0.498 g, 4.696 mmol) (DEG); dibutyl tin dilaurate (0.161 g); and 150 mL methylene chloride. The contents were refluxed. After overnight, the amount of isocyanate decreased to 43.3% as determined by titration. Then α, ω-bis(4-hydroxybutyl) polydimethylsiloxane (45.873 g, 9.557 mmol) from Example 1 was added to the flask. The refluxing was continued overnight, and no unreacted isocyanate remained as determined by titration. Then, IPDI (1.261 g, 5.673 mmol) was added and the reflux was continued overnight. The amount of isocyanate decreased to 22.9% as determined by titration. The contents were cooled down to ambient temperature. 1,1'-bi-2- naphthol (0.008 g) and 2-hydroxyethyl methacrylate (0.429 g, 3.296 mmol) were then added and the contents were stirred at ambient until isocyanate peak at 2267 $cm^{-1}$ disappeared from IR spectrum of the product (about 20 hours). The solvent was then stripped under reduced pressure and the 44.55 g of product was recovered. Theoretically, the prepolymer had 3 strong hard segments, 4 weak hard segments (x about 3, y about 4).

## Example 4

### Preparation of a polydimethylsiloxane-based prepolymer using PDMS of Example 1 and containing blocks of formulae (I) and (II)

A dry 3-neck, 500-mL round-bottom flask was connected to a nitrogen inlet tube and a reflux condenser. The following were added to the flask all at once: isophorone diisocyanate (7.825 g, 35.202 mmol) (IPDI); α, ω -bis(4-hydroxybutyl)polydimethylsiloxane (94.31 g, 19.648 mmol) from Example 1; dibutyl tin dilaurate (0.297 g); and 250 mL methylene chloride. The contents were refluxed. After overnight, the amount of isocyanate was determined to decrease to 44.5 % by titration. Then diethyleneglycol (1.421 g, 13.391 mmol) (DEG) was added to the flask. The refluxing was continued for overnight, and the amount of isocyanate was dropped down to 5.1 % of the original as determined by titration. Then the contents were cooled down to ambient temperature. 1,1'-bi-2- naphthol (0.013 g) and 2-hydroxyethyl methacrylate (0.819 g, 6.293 mmol) were then added and the contents were stirred at ambient until isocyanate peak at 2267 $cm^{-1}$ disappeared from IR spectrum of the product (about 20 hours). The solvent was then stripped under reduced pressure and the 82 g of product was recovered. Theoretically, the prepolymer had 4 strong hard segments, 3 weak hard segments.

## Example 5

### Preparation of a polydimethylsiloxane-based prepolymer using PDMS of Example 1 and containing blocks of formulae (I) and (II)

A prepolymer with components of similar molar ratios as that of Example 4 was prepared. This synthesis was similar to Example 4 except a second batch of polysiloxane of about the same molecular weight was used. The amounts of components were: isophorone diisocyanate (8.716 g, 39.209 mmol); α, ω -bis(4-hydroxybutyl)-polydimethylsiloxane (105.23 g, 21.923 mmol); dibutyl tin dilaurate (0.307 g); 250 mL methylene chloride; diethyleneglycol (1.496 g, 14.093

mmol); 1,1'-bi-2- naphthol (0.0146 g); and 2-hydroxyethyl methacrylate (1.033 g, 7.938 mmol).

**Example 6**

**Preparation of a polydimethylsiloxane-based prepolymer using PDMS of Example 2 and containing blocks of formulae (I) and (II)**

A dry 3-neck, 500 mL round-bottom flask was connected to a nitrogen inlet tube and a reflux condenser. The following were added to the flask all at once: IPDI (10.3311 g, 46.475 mmol); $\alpha,\omega$-bis(4-hydroxybutyl)polydimethylsiloxane (84.68 g, 31.023 mmol) from Example 2; dibutyl tin dilaurate (0.300 g); and 200 mL of methylene chloride. The contents were refluxed. After overnight, the amount of isocyanate was determined to decrease to 33.6 % by titration. Then, DEG (1.092 g, 10.288 mmol) was added to the flask. The refluxing was continued for 60 hours, and the amount of isocyanate was dropped down to 11.4 % of the original as determined by titration. Then, the contents were cooled down to ambient temperature. 1,1'-bi-2-naphthol (0.012 g) and 2-hydroxyethyl methacrylate (1.639 g, 12.595 mmol) were then added and the contents were stirred at ambient until isocyanate peak at 2267 $cm^{-1}$ disappeared from IR spectrum of the product (about 20 hours). The solvent was then stripped under reduced pressure to yield a clear liquid product (96.67 g). Theoretically the prepolymer has 6 PDMS block and 2 strong hard segments (x about 2, y about 5).

**Examples 7-12**

**Copolymers from prepolymer of Example 3**

Monomer mixtures were made by mixing the following components, listed in Table 1 at amounts per weight: prepolymers of Examples 3 and 4; methacryloxypropyl tris(trimethylsiloxy)silane (TRIS); N,N-dimethylacrylamide (DMA); 2-hydroxy ethyl methacrylate (HEMA); N-vinyl pyrrolidone (NVP); and methacryloxyethyl vinylcarbonate (HemaVC). Additionally, each monomer mixture included: 1,4-bis(2-methacrylamidoethylamino)anthraquinone as a tint (150 ppm); hexanol as a diluent (10 parts by weight); and Darocur-1173™ UV initiator (Ciba Specialty Chemical, Ardsley NY) (0.5 wt%).

The monomer mixtures were cast between silane-treated glass plates, and then cured under UV light for 1 hour. Each monomer mixture was cast between three sets of glass plates, each set of plates separated by Teflon™ polymer tapes of different thicknesses, such that three sets of film samples were obtained for each monomer mixture, with film thicknesses of about 200, 400 and 600 microns. The cured films were then extracted with isopropanol overnight, followed by hydration in deionized (DI) water, boiled in DI water for 4 hours and then saturated in borate buffered saline or phosphate buffered saline to give hydrogel films. The water content was measured gravimetrically. Mechanical tests were conducted in borate buffered saline according to ASTM D-1708a, discussed above. The oxygen permeabilities, reported in Dk (or barrer) units, were measured in phosphate buffered saline at 35°C, using acceptable films with three different thicknesses, as discussed above.

**Table 1**

| Example | 7 | 8 | 9 | 10 | 11 | 12 |
|---|---|---|---|---|---|---|
| Prepolymer Ex 3 | 65 | 65 | 60 | 65 | 65 | 60 |
| Tris | 10 | 10 | 15 | 10 | 10 | 15 |
| DMA | 25 | 12 | 12 | 12 | 12.4 | 0 |
| NVP | -- | 13 | 10 | 10 | 10 | 22 |
| Hema | -- | 5 | 5 | 2.65 | 2.4 | 5 |
| HemaVC | -- | 0.5 | 0.5 | 0.5 | 0.5 | 0.5 |
| % Water | 34.2 | ND | ND | 31.7 | 33.9 | 36.5 |
| Dk (barrer) | --(1) | ND | ND | 251 | 208 | 169(2) |
| Modulus ($g/mm^2$) | 45 | ND | ND | 57 | -- | -- |

The monomer mixtures prepared in Examples 8 and 9 were cloudy so no films were cast. However, when less HEMA was used as a hydrophilic comonomer (as in Examples 9, 10 and 11), or when DMA was replaced totally with NVP (as in Example 6), the mixes were clear and all hydrogel films were clear. (1) Three thickness data points were not

obtained. (2) Four thickness data points were obtained.

**Examples 13-18**

**Copolymers** from **Prepolymer** of Example 4

Following the general procedures of Examples 7-12, monomer mixtures were prepared, copolymer films were cast, and properties were evaluated, using the prepolymer of Example 4. The results are summarized in Table 2.

**Table 2**

| **Example** | **13** | **14** | **15** | **16** | **17** | **18** |
|---|---|---|---|---|---|---|
| Prepolymer Ex 4 | 65 | 65 | 60 | 65 | 60 | 65 |
| Tris | 10 | 10 | 15 | 10 | 15 | 10 |
| DMA | 25 | 12 | 12 | 12 | 0 | 0 |
| NVP | -- | 10 | 10 | 10 | 22 | 22 |
| Hema | -- | 0 | 5 | 5 | 5 | 5 |
| HemaVC | -- | 0.5 | 0.5 | 0.5 | 0.5 | 0.5 |
| % Water | 31.7 | 28.5 | 29.6 | 34.7 | 48.2 | 47.8 |
| Dk (barrer) | 158 | 208 | 218 | 209 | 215 | 183 |
| Modulus (g/mm$^2$) | 60 | 66 | 54 | 61 | 73 | 76 |

When comparing the prepolymers of Example 3 and Example 4, it was found that the prepolymer of Example 4 can be used to formulate with 5 parts of HEMA, instead of only 2.5 parts as with the prepolymer of Example 3. It is believed this is because the prepolymer of Example 4 had more strong hard segment content than the prepolymer of Example 3.

**Examples 19-24**

**Copolymers derived from Prepolymer of Example 5**

Following the general procedures of Examples 7-12, monomer mixtures were prepared, copolymer films were cast, and properties were evaluated, using the prepolymer of Example 5. The results are summarized in Table 3.

**Table 3**

| **Example** | **19** | **20** | **21** | **22** | **23** | **24** |
|---|---|---|---|---|---|---|
| Prepolymer Ex 5 | 65 | 65 | 65 | 65 | 60 | 60 |
| Tris | 10 | 10 | 10 | 10 | 15 | 15 |
| DMA | 4 | 8 | 12 | 12 | 0 | 0 |
| NVP | 18 | 14 | 10 | 10 | 22 | 25 |
| Hema | 3 | 3 | 3 | 5 | 5 | 5 |
| HemaVC | 0.9 | 0.7 | 0.5 | 0.5 | 1.0 | 0.5 |
| % Water | 26.9 | 27.8 | 28.0 | 28.8 | 26.8 | 36.4 |
| Dk (barrer) | -- | -- | -- | -- | 287 | -- |
| Modulus (g/mm$^2$) | 96 | 84 | 74 | 73 | 107 | 73 |

## Examples 25-27

### Copolymers derived from Prepolymer of Example 6

Following the general procedures of Examples 7-12, monomer mixtures were prepared, copolymer films were cast, and properties were evaluated, using the prepolymer of Example 6. The results are summarized in Table 4.

**Table 4**

| Example | 25 | 26 | 27 |
|---|---|---|---|
| Prepolymer Ex | 65 | 65 | 60 |
| Tris | 10 | 10 | 15 |
| DMA | 25 | 12 | 12 |
| NVP | -- | 10 | 10 |
| Hema | -- | 5 | 5 |
| HemaVC | -- | 0.5 | 0.5 |
| % Water | 29.8 | 23.6 | 25.8 |
| Dk (barrer) | 122 | 165 | 161 |
| Modulus (g/mm$^2$) | 81 | 119 | 84 |

## Example 28

### Preparation of α, ω-bis(4-hydroxybutyl)polydimethylsiloxane (Mn about 3600)

The following were charged to a 2-L, three-neck round-bottom flask equipped with one reflux condenser: 51.26grams of 1,3-bishydroxybutyl tetramethyldisiloxane; 863 grams of dimethoxydimethylsilane; 126 grams of distilled water; and 14.7 mL of concentrated hydrochloric acid. The mixture was heated at 60°C for 1 hour. Methanol was then distilled off over a 5-hour period. Then, 279 ml distilled water and 279 mL concentrated HCl were added, and the contents were refluxed at 100°C for 3 hours. The crude product was then separated from the aqueous layer. Then, 600 mL diethyl ether and 400 mL distilled water were added, and the solution was extracted twice with 400 mL sodium bicarbonate solution (0.5 %) and then with distilled water until the washing had neutral pH. The product was then added slowly into a mixture of methanol/water (406g/118g). The bottom organic layer was separated, added with diethyl ether and dried with magnesium sulfate. Ether was then stripped under vacuum at room temperature and the residue was further stripped under vacuum (0.07-mm torr) at 80° C. The final product was recovered. The molecular weight (Mn) as determined by titration was 3598.

## Example 29

### Preparation of α,ω-polydimethylsiloxane prepolymer using PDMS of Example 28 and having formula (V)

A dry 3-neck, 500-mL round-bottom flask was connected to a nitrogen inlet tube and a reflux condenser. The following were added to the flask all at once: isophorone diisocyanate (9.188 g, 41.333 mmol) (IPDI); α ,ω-bis(4-hydroxybutyl)-polydimethylsiloxane from Example 1 (114.68 g, 31.873 mmol) (PDMS); dibutyl tin dilaurate (0.327 g); and 180 mL methylene chloride. The contents were refluxed. After overnight, the amount of isocyanate was determined to decrease to 22.0 % by titration. The contents were cooled down to ambient temperature. 1,1'-bi-2- naphthol (0.0144 g) and 2-hydroxyethyl methacrylate (2.915 g, 22.399 mmol) were then added and the contents were stirred at ambient until isocyanate peak at 2267 cm$^{-1}$ disappeared from IR spectrum of the product. The solvent was then stripped under reduced pressure and the product was recovered (126 g). Theoretically, the prepolymer had 3 blocks containing of PDMS (x about 3).

**Example 30**

**Preparation of α,ω-polydimethylsiloxane prepolymer using PDMS of Example 28 and having formula (V)**

The general procedure of Example 29 is followed, except that the molar ratio of PDMS to IPDI is 4:5, respectively. 149.6 g of prepolymer was recovered. Theoretically, the prepolymer had 4 blocks containing of PDMS (x about 4).

**Example 31**

**Preparation of α,ω-polydimethylsiloxane prepolymer using PDMS of Example 28 and having formula (V)**

The general procedure of Example 29 is followed, except that the molar ratio of PDMS to IPDI is 5:6, respectively. 159.9 g of prepolymer was recovered. Theoretically, the prepolymer had 5 blocks containing of PDMS (x about 5).

**Examples 32-41**

**Copolymers**

Monomer mixtures were made by mixing the following components, listed in Tables 5 and 6 at amounts per weight: prepolymers of Examples 29, 30, or 31; methacryloxypropyl tris(trimethylsiloxy)silane (TRIS); N,N-dimethylacrylamide (DMA); 2-hydroxy ethyl methacrylate (HEMA); N-vinyl pyrrolidone (NVP); and/or methacryloxyethyl vinylcarbonate (HemaVC). Additionally, each monomer mixture included: 1,4-bis(2-methacrylamidoethylamino)anthraquinone as a tint (150 ppm); hexanol as a diluent (10 parts by weight); and Daroctir™ UV initiator (Ciba Specialty Chemical, Ardsley NY) (0.5 wt%). The monomer mixtures were cast and cured into films following the general procedure of Examples 7-12.

**Table 5**

| Example | 32 | 33 | 34 | 35 | 36 |
|---|---|---|---|---|---|
| Prepolymer Ex 29 | 65 | 65 | 60 | 65 | -- |
| Prepolymer Ex 30 | -- | -- | -- | -- | 65 |
| Tris | 10 | 10 | 15 | 10 | 10 |
| DMA | 15 | 12 | 12 | 25 | 25 |
| NVP | 10 | 10 | 10 | -- | -- |
| Hema | -- | 5 | 5 | -- | -- |
| HemaVC | 0.5 | 0.5 | 0.5 | -- | -- |
| % Water | 19.6 | 18.4 | 19.1 | 19.3 | 22.3 |
| Dk (barrer) | 224 | 300 | 224 | 219 | 257 |
| Modulus ($g/mm^2$) | 187 | 180 | 143 | 152 | 102 |

**Table 6**

| Example | 37 | 38 | 39 | 40 | 41 |
|---|---|---|---|---|---|
| Prepolymer Ex 30 | 65 | 60 | -- | -- | -- |
| Prepolymer Ex 31 | -- | -- | 65 | 65 | 60 |
| Tris | 10 | 15 | 10 | 10 | 15 |
| DMA | 12 | 12 | 25 | 12 | 15 |
| NVP | 10 | 10 | -- | 5 | 10 |
| Hema | 5 | 5 | -- | -- | 2 |
| HemaVC | 0.5 | 0.5 | -- | 0.5 | 0.5 |

(continued)

| Example | 37 | 38 | 39 | 40 | 41 |
|---|---|---|---|---|---|
| % Water | ND | ND | 25.9 | ND | 23.9 |
| Dk (barrer) | ND | ND | 171 | ND | 159 |
| Modulus (g/mm$^2$) | ND | ND | 85 | ND | 79 |

The monomer mixtures prepared in Examples 37, 38 and 40 were cloudy so no films were cast. As the prepolymer in these examples were less polar, this suggests that prepolymers of lower polarity are less compatible with hydrophilic monomers. All hydrogel films were optically clear.

As seen in Figure 2, the claimed copolymers have Dk/water content values falling well above the line represented various prior silicone hydrogel copolymers, and the majority of these copolymers have desirable tensile modulus for soft contact lens applications. Several additional observations are noted. First, copolymers including a meth(acrylic) substituted alcohol tended to have higher oxygen permeability for a given water content. Second, copolymers having a prepolymer with a silicon atom content of at least 30 weight percent tended to have a higher oxygen permeability for a given water content.

**Example 42**

**Preparation of a polydimethylsiloxane-based prepolymer using PDMS of Example 1 and containing blocks of formulae (I) and (II)**

A prepolymer was prepared following the general synthesis of Example 4. The amounts of components were: isophorone diisocyanate (6.650 g, 29.918 mmol); α, ω -bis(4-hydroxybutyl)-polydimethylsiloxane (72.71 g, 15.148 mmol); dibutyl tin dilaurate (0.215 g); 200 mL methylene chloride; diethyleneglycol (1.186 g, 11.172 mmol); 1,1'-bi-2- naphthol (0.012 g); and 2-hydroxyethyl methacrylate (0.986 g, 7.576 mmol).

**Example 43**

**Preparation of a polydimethylsiloxane-based prepolymer using PDMS of Example 1 and containing blocks of formulae (I) and (II)**

A prepolymer was prepared following the general synthesis of Example 4. The amounts of components were: isophorone diisocyanate (12.370 g, 55.649 mmol); α, ω -bis(4-hydroxybutyl)-polydimethylsiloxane (133.47 g, 27.806 mmol); dibutyl tin dilaurate (0.404 g); 300 mL methylene chloride; diethyleneglycol (2.257 g, 21.270 mmol); 1,1'-bi-2-naphthol (0.021 g); and 2-hydroxyethyl methacrylate (1.678 g, 12.894 mmol).

**Example 44**

**Preparation of α, ω-bis(4-hydroxybutyl)polydimethylsiloxane (Mn about 4000)**

The general procedure of Example 1 was followed for making this polysiloxane, except the molar ratio of 1,3-bishydroxybutyl tetramethyldisiloxane to dimethoxydimethylsilane was changed to about 1:45. The molecular weight (Mn) of the product as determined by titration was 4000.

Preparation of a polydimethylsiloxane-based prepolymer using PDMS of Example 44

A prepolymer was prepared following the general synthesis of Example 4 and employing the PDSM of this example. The amounts of components were: isophorone diisocyanate (23.149g, 104.139 mmol); α, ω-bis(4-hydroxybutyl)-polydimethylsiloxane (208.54 g, 51.568 mmol); dibutyl tin dilaurate (0.690 g); 400 ml methylene chloride; diethyleneglycol (4.769 g, 44.940 mmol); 1,1'-bi-2-naphthol (0.032 g); and 2-hydroxyethyl methacrylate (1.751 g, 13.455 mmol).

**Examples 45-52**

**Copolymers**

Monomer mixtures were made by mixing the following components, listed in Tables 7 and 8 at amounts per weight: prepolymers of Examples 42, 43 or 44; methacryloxypropyl tris(trimethylsiloxy)silane (TRIS); N,N-dimethylacrylamide (DMA); 2-hydroxy ethyl methacrylate (HEMA); N-vinyl pyrrolidone (NVP); and/or methacryloxyethyl vinylcarbonate (HemaVC). Additionally, each monomer mixture included: 1,4-bis(2-methacrylamidoethylamino)anthraquinone as a tint (150 ppm); and Darocur™ UV initiator (Ciba Specialty Chemical, Ardsley NY) (0.5 wt%). The monomer mixtures were cast and cured into films following the general procedure of Examples 7-12. The Dk values in Tables 7 and 8 were derived from five data points, except for Example 51 which was derived from four data points.

**Table 7**

| Example | 45 | 46 | 47 | 48 |
|---|---|---|---|---|
| Prepolymer Ex 42 | 40 | 40 -- | | -- |
| Prepolymer Ex 43 | -- | -- | 40 | 40 |
| Tris | 20 | 20 | 20 | 20 |
| DMA | 10 | 10 | 10 | 10 |
| NVP | 30 | 30 | 30 | 30 |
| Hema | 0 | 3 | 0 | 3 |
| HemaVC | 1 | 0.7 | 0.7 | 0.7 |
| n-hexanol | 10 | 10 | 10 | 10 |
| % Water | 47 | 47 | 49 | 48 |
| Dk (barrer) | 116 | 97 | 101 | 100 |
| Modulus (g/mm$^2$) | 56 | 55 | 51 | 54 |

**Table 8**

| Example | 49 | 50 | 51 | 52 |
|---|---|---|---|---|
| Prepolymer Ex 43 | -- | 40 | 40 | 40 |
| Prepolymer Ex 44 | 40 | -- | -- | -- |
| Tris | 18 | 20 | 10 | 17 |
| DMA | 9 | 10 | 10 | 9 |
| NVP | 40 | 40 | 33 | 35 |
| HemaVC | 0.8 | 0.8 | 0.7 | 0.7 |
| n-hexanol | 10 | 15 | 16 | 15 |
| % Water | 60 | 54 | 52 | 53 |
| Dk (barrer) | 74 | 83 | 80 | 85 |
| Modulus (g/mm$^2$) | 43 | 57 | 57 | 56 |

Having thus described various preferred embodiment of the invention, those skilled in the art will appreciate that various modifications, additions, and changes may be made thereto without departing from the scope of the invention, as set forth in the following claims.

## Claims

1. A hydrogel copolymer that is the hydrated polymerization product of a monomeric mixture comprising a polysiloxane prepolymer comprising soft segments that are a diradical residue of the formula (PS'):

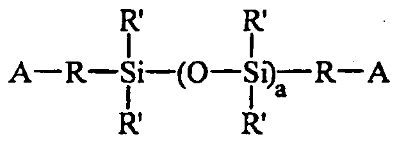

(PS')

wherein:

each A is a hydroxyl or an amino radical;
each R is independently selected from an alkylene group having 1 to 10 carbon atoms wherein the carbon atoms may include ether, urethane or ureido linkages therebetween;
each R' is independently selected from hydrogen, monovalent hydrocarbon radicals or halogen substituted monovalent hydrocarbon radicals wherein the hydrocarbon radicals have 1 to 20 carbon atoms which may include ether linkages therebetween, and
a is at least 1,
wherein the silicon atom content of the prepolymer is at least 32 weight % of the prepolymer;
a monofunctional silicone-containing monomer; and
a hydrophilic comonomer selected from the group consisting of: methacrylic acid; acrylic acid; 2-hydroxyethylmethacrylate; N-vinyl pyrrolidone; and methacrylamide; the hydrogel copolymer having a water content in the range of 40 to 50 weight percent, and an oxygen permeability greater than 100 barrers.

2. The hydrogel copolymer of claim 1, having either:

a water content in the range of 40 to no greater than 45 weight percent; or
a water content in the range of 45 to 50 weight percent.

3. The hydrogel copolymer of claim 1, having a modulus between 40 and 80 g/mn$^2$.

4. The hydrogel copolymer of claim 1, wherein the silicon atom content of the prepolymer is at least 33 weight % of the prepolymer.

5. The hydrogel copolymer of claim 1, wherein the prepolymer has a molecular weight (Mn) of at least 10,000, or at least 15,000.

6. The hydrogel copolymer of claim 1, wherein the monomeric mixture includes 2-hydroxyethylmethacrylate.

7. The hydrogel copolymer of claim 6, wherein the monomeric mixture further comprises N-vinyl pyrrolidone.

8. The hydrogel copolymer of claim 1, wherein each R is alkylene, and each R' is independently alkyl or fluoroalkyl optionally including ether linkages.

9. The hydrogel copolymer of claim 1, wherein the prepolymer further comprises at least one member selected from the group consisting of: strong hard segments represented by *Dii*Diol*Dii*; and weak hard segments represented by *Dii*; wherein each Dii is independently a diradical residue of a diisocyanate, each Diol is independently a diradical residue of a diol having 1 to 10 carbon atoms, and each * is independently -NH-CO-NH-, -NH-COO- or -OCONH-.

10. The hydrogel copolymer of claim 9, wherein the prepolymer comprises said soft segments and said strong hard segments, represented by *Dii*Diol*Dii*PS*Dii* wherein PS is the diradical residue of formula (PS').

11. The hydrogel copolymer of claim 10, wherein the weight ratio of said soft segments to said strong hard segments is at least 5:1 and no greater than 13:1.

**12.** The hydrogel copolymer of claim 1, wherein the prepolymer is selected from one of the following formulae:

$M(*Dii*Diol*Dii*PS)_x(*Dii*PS)y*Dii*M$
$M(*Dii*Diol*Dii*PS)_x(*Dii*PS)_y*Dii*Diol*Dii*M$
$M(*Dii*PS*Dii*Diol)_x*Dii*PS*Dii*M$
$M(*Dii*Diol*Dii*PS)_x*Dii*Diol*Dii*M$
$M(*Dii*PS)_x*Dii*M$
wherein:

each M is independently a polymerizable ethylenically unsaturated radical;
each Dii is independently a diradical residue of a diisocyanate;
each Diol is independently a diradical residue of a diol having 1 to 10 carbon atoms;
each PS is independently a diradical residue of a polysiloxane-diol;
each * is independently -NH-CO-NH- or -NH-COO- x is at least 2, and
y is at least 1.

**13.** A contact lens made of the hydrogel copolymer of claim 1, 2 or 3.

**Patentansprüche**

**1.** Ein Hydrogel-Copolymer, welches das hydratisierte Polymerisationsprodukt von einem Monomerengemisch, umfassend
ein Polysiloxan-Vorpolymer, umfassend weiche Segmente, welche ein zweiwertiger Rest der Formel (PS') sind:

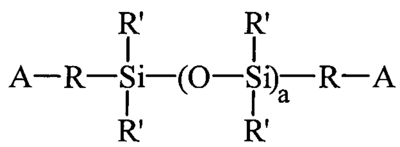
(PS')

wobei:

jedes A ein Hydroxyl- oder ein Aminorest ist;
jedes R unabhängig ausgewählt ist aus einem Alkylenrest mit 1 bis 10 Kohlenstoffatomen, wobei die Kohlenstoffatome Ether-, Urethan- oder Ureidoverknüpfungen dazwischen enthalten können;
jedes R' unabhängig ausgewählt ist aus Wasserstoff, einwertigen Kohlenwasserstoffresten oder Halogen-substituierten einwertigen Kohlenwasserstoffresten, wobei die Kohlenwasserstoffreste 1 bis 20 Kohlenstoffatome aufweisen, welche Etherverknüpfungen dazwischen enthalten können; und
a mindestens 1 ist,
wobei der Siliziumatomgehalt des Vorpolymers mindestens 32 Gewichtsprozent des Vorpolymers beträgt;
ein monofunktionelles Silikon enthaltendes Monomer; und
ein hydrophiles Comonomer, ausgewählt aus der Gruppe bestehend aus: Methacrylsäure, Acrylsäure, 2-Hydroxyethylmethacrylat, N-Vinylpyrrolidon und Methacrylamid; ist, wobei das Hydrogel-Copolymer einen Wassergehalt in dem Bereich von 40 bis 50 Gewichtsprozent und eine Sauerstoffpermeabilität von größer als 100 Barrer aufweist.

**2.** Das Hydrogel-Copolymer gemäß Anspruch 1, wobei es entweder:

einen Wassergehalt in dem Bereich von 40 bis nicht größer als 45 Gewichtsprozent; oder
einen Wassergehalt in dem Bereich von 45 bis 50 Gewichtsprozent aufweist.

**3.** Das Hydrogel-Copolymer gemäß Anspruch 1 mit einem Modul zwischen 40 und 80 $g/mm^2$.

**4.** Das Hydrogel-Copolymer gemäß Anspruch 1, wobei der Siliziumatomgehalt des Vorpolymers mindestens 33 Gewichtsprozent des Vorpolymers beträgt.

5. Das Hydrogel-Copolymer gemäß Anspruch 1, wobei das Vorpolymer ein Molekulargewicht (Mn) von mindestens 10.000 oder mindestens 15.000 aufweist.

6. Das Hydrogel-Copolymer gemäß Anspruch 1, wobei das Monomerengemisch 2-Hydroxyethylmethacrylat enthält.

7. Das Hydrogel-Copolymer gemäß Anspruch 6, wobei das Monomerengemisch ferner N-Vinylpyrrolidon umfasst.

8. Das Hydrogel-Copolymer gemäß Anspruch 1, wobei jedes R Alkylen ist, und jedes R' unabhängig Alkyl oder Fluoralkyl, das gegebenenfalls Etherverknüpfungen enthält, ist.

9. Das Hydrogel-Copolymer gemäß Anspruch 1, wobei das Vorpolymer ferner mindestens einen Bestandteil, ausgewählt aus der Gruppe bestehend aus: starken harten Segmenten, dargestellt durch *Dii*Diol*Dii*; und schwachen harten Segmenten dargestellt durch *Dii* umfasst; wobei jedes Dii unabhängig ein zweiwertiger Rest eines Diisocyanats ist, jedes Diol unabhängig ein zweiwertiger Rest eines Diols mit 1 bis 10 Kohlenstoffatomen ist, und jedes * unabhängig -NH-CO-NH-, -NH-COO- oder -OCONH- ist.

10. Das Hydrogel-Copolymer gemäß Anspruch 9, wobei das Vorpolymer die weichen Segmente und die starken harten Segmente umfasst, dargestellt durch *Dii*Diol*Dii*PS*Dii*, wobei PS der zweiwertige Rest von Formel (PS') ist.

11. Das Hydrogel-Copolymer gemäß Anspruch 10, wobei das Gewichtsverhältnis der weichen Segmente zu den starken harten Segmenten mindestens 5:1 und nicht größer als 13:1 ist.

12. Das Hydrogel-Copolymer gemäß Anspruch 1, wobei das Vorpolymer ausgewählt ist aus einer der folgenden Formeln:

M(*Dii*Diol*Dii*PS)$_x$(*Dii*PS)$_y$*Dii*M
M(*Dii*Diol*Dii*PS)$_x$(*Dii*PS)$_y$*Dii*Diol*Dii*M
M(*Dii*PS*Dii*Diol)$_x$*Dii*PS*Dii*M
M(*Dii*Diol*Dii*PS)$_x$*Dii*Diol*Dii*M
M(*Dii*PS)$_x$*Dii*M
wobei:

jedes M unabhängig ein polymerisierbarer ethylenisch ungesättigter Rest ist;
jedes Dii unabhängig ein zweiwertiger Rest eines Diisocyanats ist;
jedes Diol unabhängig ein zweiwertiger Rest eines Diols mit 1 bis 10 Kohlenstoffatomen ist;
jedes PS unabhängig ein zweiwertiger Rest eines Polysiloxandiols ist;
jedes * unabhängig -NH-CO-NH- oder -NH-COO- ist;
x mindestens 2 ist, und
y mindestens 1 ist.

13. Eine Kontaktlinse, hergestellt aus dem Hydrogel-Copolymer gemäß Anspruch 1, 2 oder 3.

## Revendications

1. Hydrogel copolymère qui est le produit de polymérisation hydraté d'un mélange monomère comprenant un prépolymère de polysiloxane comprenant des segments souples qui sont un résidu diradicalaire répondant à la formule (PS') :

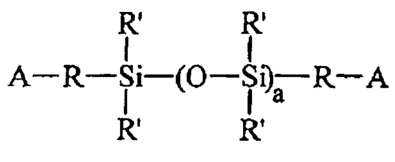

(PS')

dans laquelle :

chaque radical A représente un radical hydroxyle ou amino ;
chaque radical R est indépendamment choisi parmi un groupe alkylène comportant de 1 à 10 atomes de carbone dans lequel les atomes de carbone peuvent comprendre des liaisons éther, uréthane ou uréido entre ceux-ci ;
chaque radical R' est indépendamment choisi parmi un atome d'hydrogène, des radicaux hydrocarbure monovalents ou des radicaux hydrocarbure monovalents substitués par des atomes d'halogène dans lequel les radicaux hydrocarbure comportent de 1 à 20 atomes de carbone qui peuvent comprendre des liaisons éther entre ceux-ci, et
a vaut au moins 1,
dans lequel la teneur en atomes de silicium du prépolymère vaut au moins 32 % en poids du prépolymère ;
un monomère contenant de la silicone monofonctionnelle ; et
un comonomère hydrophile choisi dans le groupe constitué : de l'acide méthacrylique ; de l'acide acrylique ; du 2-hydroxyéthylméthacrylate ; de la N-vinylpyrrolidone ; et du méthacrylamide ; l'hydrogel copolymère ayant une teneur en eau dans la plage de 40 à 50 pour cent en poids, et une perméabilité à l'oxygène supérieure à 100 barrer.

2. Hydrogel copolymère selon la revendication 1, ayant soit :

une teneur en eau dans la plage de 40 à pas plus de 45 pour cent en poids ; ou
une teneur en eau dans la plage de 45 à 50 pour cent en poids.

3. Hydrogel copolymère selon la revendication 1, ayant un module entre 40 et 80 g/mm$^2$.

4. Hydrogel copolymère selon la revendication 1, dans lequel la teneur en atomes de silicium du prépolymère vaut au moins 33 % en poids du prépolymère.

5. Hydrogel copolymère selon la revendication 1, dans lequel le prépolymère a un poids moléculaire (Mn) d'au moins 10 000, ou d'au moins 15 000.

6. Hydrogel copolymère selon la revendication 1, dans lequel le mélange monomère comprend du 2-hydroxyéthylméthacrylate.

7. Hydrogel copolymère selon la revendication 6, dans lequel le mélange monomère comprend en outre de la N-vinylpyrrolidone.

8. Hydrogel copolymère selon la revendication 1, dans lequel chaque radical R représente un groupe alkylène, et chaque radical R' représente indépendamment un groupe alkyle ou fluoroalkyle comprenant optionnellement des liaisons éther.

9. Hydrogel copolymère selon la revendication 1, dans lequel le prépolymère comprend en outre au moins un élément choisi dans le groupe constitué : des segments durs et résistants représentés par *Dii*Diol*Dii* ; et des segments durs et fragiles représentés par *Dii* ; formules dans lesquelles chaque Dii représente indépendamment un résidu diradicalaire d'un diisocyanate, chaque Diol représente indépendamment un résidu diradicalaire d'un diol comportant de 1 à 10 atomes de carbone, et chaque * représente indépendamment -NH-CO-NH-, -NH-COO- ou -OCONH-.

10. Hydrogel copolymère selon la revendication 9, dans lequel le prépolymère comprend lesdits segments souples et lesdits segments durs et résistants, représentés par la formule *Dii*Diol*Dii*PS*Dii* dans laquelle PS représente le résidu diradicalaire de formule (PS').

11. Hydrogel copolymère selon la revendication 10, dans lequel le rapport pondéral desdits segments souples auxdits segments durs et résistants vaut au moins 5/1 et pas plus de 13/ 1.

12. Hydrogel copolymère selon la revendication 1, dans lequel le prépolymère est choisi parmi l'une des formules suivantes :

$$M(*Dii*Diol*Dii*PS)_x(*Dii*PS)_y*Dii*M$$

M(*Dii*Diol*Dii*PS)$_x$(*Dii*PS)$_y$*Dii*Diol*Dii*M
M(*Dii*PS*Dii*Diol)$_x$*Dii*PS*Dii*M
M(*Dii*Diol*Dii*PS)$_x$*Dii*Diol*Dii*M
M(*Dii*PS)$_x$*Dii*M
dans lesquelles :

chaque M représente indépendamment un radical éthyléniquement insaturé polymérisable ;
chaque Dii représente indépendamment un résidu diradicalaire d'un diisocyanate ;
chaque Diol représente indépendamment un résidu diradicalaire d'un diol comportant de 1 à 10 atomes de carbone ;
chaque PS représente indépendamment un résidu diradicalaire d'un polysiloxane-diol ;
chaque * représente indépendamment -NH-CO-NH- ou -NH-COO;
x vaut au moins 2, et
y vaut au moins 1.

**13.** Lentille de contact fabriquée à partir de l'hydrogel copolymère selon la revendication 1, 2 ou 3.

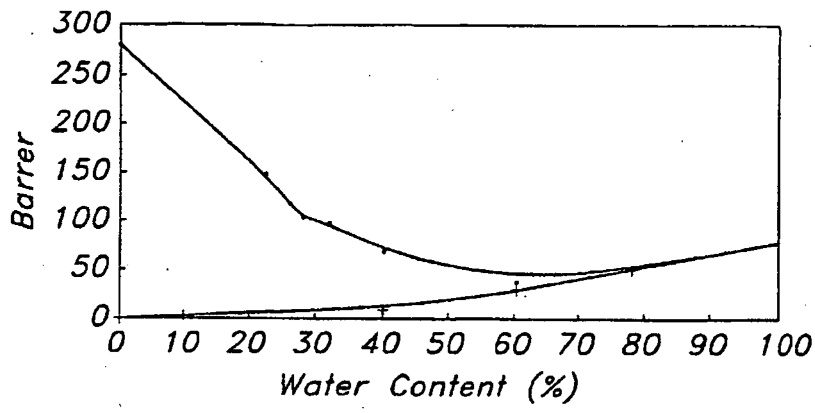
300
250
200
150
100
50
0
Barrer
0 10 20 30 40 50 60 70 80 90 100
Water Content (%)

FIG. 1

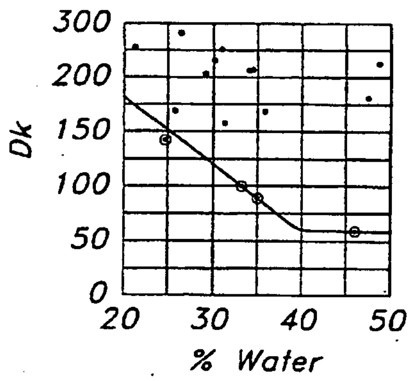
300
200
150
100
50
0
Dk
20 30 40 50
% Water

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003162862 A1
- US 3408429 A
- US 3660545 A
- US 4113224 A
- US 4197266 A
- US 5271875 A
- US 5260000 A
- US 5034461 A, Lai